# EUROPEAN PATENT SPECIFICATION

(11) **EP 4 341 445 B1**
(45) Date of publication and mention of the grant of the patent: **01.07.2026**
(21) Application number: 22805719.6
(22) Date of filing: 19.05.2022
(51) Int. Cl.: A01K 15/02, A23K 10/26, A23K 20/163, C14C 1/00, A23K 40/30, A23K 50/42, A61K 8/73, A61K 8/98, A61Q 11/00

(54) **METHOD OF FORMING A RAWHIDE ANIMAL CHEW INCLUDING MICROPORES FILLED WITH CHITOSAN**
VERFAHREN ZUR HERSTELLUNG EINES ROHHAUT-KAUARTIKELS MIT MIT CHITOSAN GEFÜLLTEN MIKROPOREN
PROCÉDÉ DE FABRICATION D'UN ARTICLE À MÂCHER POUR ANIMAUX À BASE DE PEAU BRUTE COMPRENANT DES MICROPORES REMPLIS DE CHITOSANE

(30) Priority: 20.05.2021 US 202163201970 P
(43) Date of publication of application: 27.03.2024
(73) Proprietor: IMS Trading, LLC, Jamesburg NJ 08831 (US)
(72) Inventor: AXELROD, Glen S., Colts Neck, New Jersey 07722 (US); GAJRIA, Ajay, Monmouth Junction, New Jersey 08852 (US)
(74) Representative: Zimmermann & Partner Patentanwälte mbB
(86) International application number: PCT/US2022/072419
(87) International publication number: WO 2022/246439

(56) References cited:
- EP-A2- 2 548 436
- CN-A- 101 352 583
- CN-A- 112 369 499
- CN-U- 206 538 529
- JP-A- H06 279 800
- US-A1- 2004 156 883
- US-A1- 2005 145 193
- US-A1- 2005 147 719
- US-A1- 2018 168 127
- US-A1- 2018 168 127

## Description

### FIELD

The present disclosure is directed to a rawhide animal chew including micropores filled with chitosan.

### BACKGROUND

Animal chews are commonly provided to domesticated pets, and particularly dogs, to satisfy an innate need to chew. Animal chews also provide a source of entertainment and, depending on the material which the animal chew is formed from, can provide a source of nutrients. Further, it is believed that chewing on objects, such as animal chews, provides sufficient abrasion to improve the dental health and hygiene of an animal. Rawhide pet chews are sometimes perceived as a more natural product compared to pet chews formed from thermoplastic materials. Depending on preparation, rawhide also tends to be rather durable. Due to its durability, dogs may chew on rawhide for extended time periods slowly wearing away the rawhide. However, while dogs may gnaw on rawhide for extended periods of time, there are times relatively large pieces are pull off by the dog and ingested. It is, therefore, also beneficial to provide readily digestible rawhide chews without sacrificing durability.

Various means to solve the problem of improving digestibility, while maintaining chewability and durability, has been considered in the art. For example, U.S. 8,613,261 to Mendal, et al., described treating rawhide with various enzymes to break the rawhide down to increase its digestibility. U.S. 7,691,426 to Axelrod, et al., described comminuting rawhide into small particles or powder and incorporating it into edible resin. U.S. 7,678,402 to Marino describes forming large perforations in the chew to provide small sections that break off when chewed. Further, U.S. 7,147,888 to Brown, et al., describes impregnating rawhide pet chews with biofilm disrupting emulsions.
Document US 2018/168127 A1 describes a microporous animal chew having a plurality of micropores extending through said rawhide sheet. A support additive may be retained in said micropores.
Document EP 2 548 436 A2 describes a multifunctional chewing gum for a pet. For the purpose of antibacterial activity and inflammation prevention, chitosan, a green tea extract or a mixture thereof may be used in an amount of 0.01 - 10 wt%. In one example, 6.0 wt% of gelatin, 0.8 wt% of vitamin B6 , 0.8 wt% of calcium gluconate, 0.8 wt% of chitosan and 3.0 wt% of chicken powder were mixed with a remainder of purified water to prepare a coating solution.
To assist in impregnation of the rawhide, the rawhide is perforated with slits and holes. Brown, et al., also describes that increasing the surface area to volume ratio, increases the area contacted by the digestive juices of the animal, with the goal of improving digestion. Nevertheless, the problem of providing a digestible yet chewable and durable animal chew remains.

### SUMMARY

The invention is set out in the appended set of claims.

An aspect of the present disclosure relates to a microporous animal chew. The chew includes a rawhide sheet having a thickness in the range of 0.1 millimeters to 3.0 millimeters and a plurality of micropores extending through the rawhide sheet. The micropores exhibit a longest linear dimension across a cross-section of the micropores in the range of 1 micrometer to 2,000 micrometers and are present at a density of 1 to 100 pores per square centimeter. The micropores are at least partially filled with chitosan.

Another aspect of the present disclosure relates to a method of forming a microporous animal chew. The method includes providing a wet rawhide sheet having a thickness in the range of 0.1 millimeters to 4.0 millimeters including water present 60 % to 80 % by weight of the total weight of the rawhide sheet or greater. The rawhide sheet is then pierced with pins and micropores are formed in the rawhide sheet. The micropores have a largest linear cross-sectional length in the range of 1 micrometer to 2,000 micrometers and are arranged to provide a pore density in the range of 1 to 100 pores per square centimeter. The rawhide sheet is dried to include 1 to 20 % by weight water of the total weight of the rawhide sheet. The micropores are then at least partially filled with chitosan.

### BRIEF DESCRIPTION OF THE DRAWINGS

The above-mentioned and other features of this disclosure, and the manner of attaining them, will become more apparent and better understood by reference to the following description of embodiments described herein taken in conjunction with the accompanying drawings, wherein:
FIG. 1 illustrates a method of forming an animal chew including a plurality of micropores;
FIG. 2A illustrates an embodiment of a cross-section of a micropore forming pin;
FIG. 2B illustrates an embodiment of a pore formed by the micropore forming pin exhibiting a longest linear cross-sectional length, CL;
FIG. 3A illustrates an embodiment of a press plate arrangement for forming micropores in a rawhide sheet;
FIG. 3B illustrates an embodiment of a press plate arrangement for forming micropores in a rawhide sheet;
FIG. 4 illustrates an embodiment of a calender roll system for forming micropores in a rawhide sheet; and
FIG. 5 illustrates pores formed in a rawhide sheet at an angle to the sheet surface.

### DETAILED DESCRIPTION

The present disclosure is directed to the provision of a rawhide animal chew including micropores at least partially filled with chitosan and a method of forming such an animal chew. The micropores increase the surface area to volume ratio of the rawhide, allowing for a larger surface area to be contacted with digestive juices of the animal, increasing enzymatic digestion of the animal chew. The micropores may be formed by a number of methods, including piercing rawhide sheets with pins while the rawhide is wet.

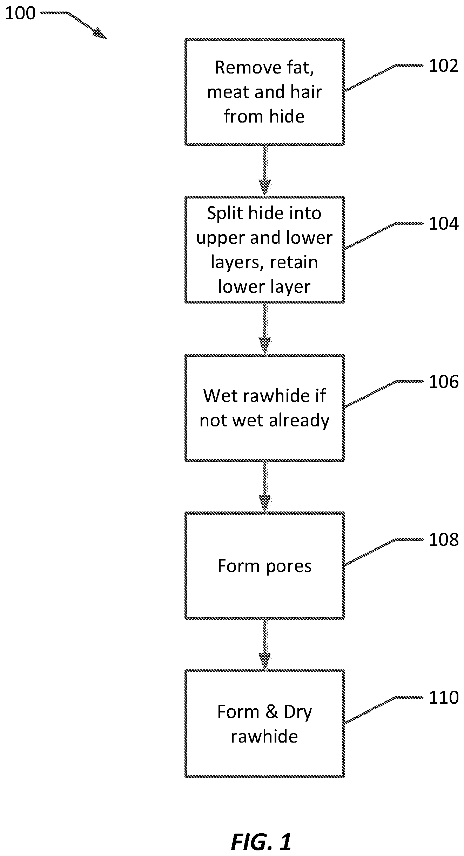

Rawhide, as referred to herein, is the untanned skin of buffalo, deer, elk, moose, cattle, pig, sheep, goats, or other hoofed animals. Generally, hides initially contain between 60 % to 80 % by weight water, such as between 60 to 70 % by weight water, and 20 % to 40 % by weight other substances such as fibrous proteins, collagen, keratin, elastin and reticulin as well as 0.01 to 2 % by weight ash including phosphorous, potassium, sodium arsenic, magnesium and calcium. The sheet containing such water has a thickness in the range of 0.1 mm to 4.0 mm. A hide may be prepared by any method known to those of ordinary skill in the art. One such method 100 is illustrated in FIG. 1 and includes removing most of the visible fat, meat and hair from the hide 102. The fat and meat may be removed by scraping and, once the fat and meat are removed, the hide may be treated in a solution of calcium carbonate, calcium hydroxide, slaked lime and wood ash, which may loosen and aid in the removal of hair. In addition, sodium sulphide, ammonium salts or enzymes may be added to the solution. However, in some preferred embodiments, enzymes and enzymatic treatments may be excluded. The hair is removed from the hide and the hide is rinsed. The hide may then be soaked in an aqueous solution including organic acids, inorganic acids and/or acid salts, such as potassium hydrogen tartrate and sodium bicarbonate. In embodiments, the hide is split 104 where the upper layer is separated from the under layer and the underlayer is used to make chews. In addition, the pieces may be soaked in a solution including hydrogen peroxide and chlorine. The hide may be rinsed again. The pieces of rawhide may be dried or further processed before drying. After drying, the hide preferably exhibits a moisture content in the range of 1 % to 20 % by weight water, including all values and ranges therein and preferably in the range of 5 % to 18 % by weight water. At such lower water levels the sheet has a preferred thickness of 0.1 mm to 3.0 mm.

Alternatively, the rawhide is provided in a rawhide resin composition, wherein the rawhide may be chopped or ground into small particles or powder. The particle size may be less than about 10 mm, such as in the range of 0.001 to 10 mm, including all values and increments therein. The moisture content of the rawhide may be adjusted to approximately 1 % to 20% by weight of the rawhide, including all increments and values therein, such at 8%, 10%, etc. The rawhide may then be combined with up to 20% by weight of casein, such as in the range of about 0.1 to 20% by weight, including all values and increments therein. Casein may be understood as a phosphoprotein of milk, wherein a phosphoprotein may be described as a group of substances that are chemically bonded to a substance containing phosphoric acid. The rawhide may also be combined with gelatin up to 10% by weight, such as in the range of 0.1 to 10% by weight, including all values and increments therein. Gelatin may be understood as a protein product produced by partial hydrolysis of collagen. In addition, attractants, such as flavorants, or nutrients may be compounded with the rawhide.

The rawhide particles, casein, gelatin and any additional attractants or nutrients may be melt processed, wherein the particles are plasticated in a plasticating device. Suitable plasticating devices may include injection molding machines, extruders (twin-screw, single screw, etc.) or any other device which may provide sufficient thermal-mechanical interaction to cause plastication, such as blenders. The temperature of the plasticating device may be sufficient to melt at least 10% to 100% of the particles, including all values and increments therein and may be in the range of about 120 to 150° C., including all values and increments therein. In addition, the particles may be pressurized during plastication wherein the applied pressure may be in the range of about 1 to 20 MPa, including all values and increments therein.

The rawhide (the rawhide pieces or rawhide resin composition) is preferably provided in the form of sheets. Prior to drying, and while wet (e.g. at a water level of greater than or equal to 60%), the rawhide sheets preferably exhibit a thickness in the range of 0.5 mm to 4 mm, including all values and ranges therein. The dried sheets (e.g. at a water content of 1% to 20% by weight) therefore have a relatively lower thickness, in the range of 0.1 millimeters to 3.0 millimeters, including all values and ranges therein, and preferably in the range of 1.0 mm to 2.0 mm or even more preferably 0.1 to 0.5 mm. Referring back to FIG. 1, the pores are preferably formed in the rawhide while it is wet.

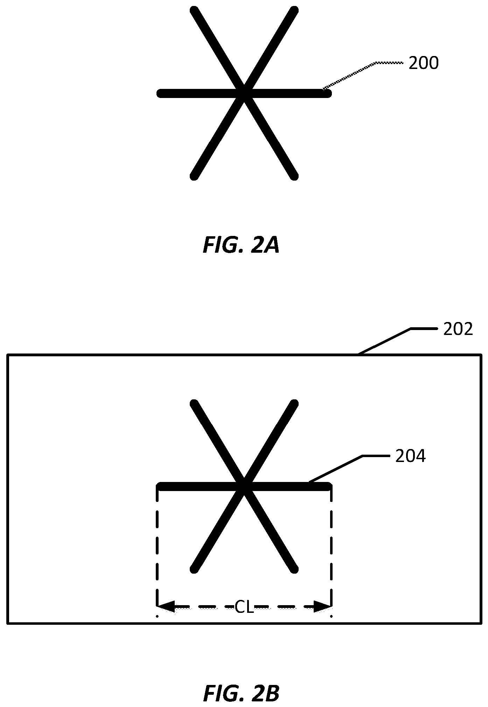

The rawhide is then pierced to form the micropores 108. Piercing may be facilitated by forcing a plurality of pins through the rawhide using, for example, a press or calendaring rolls. The micro-sized pins provide micropores having a largest linear cross-sectional length, CL, (see FIG. 2b) in the range of 1 micrometer to 2000 micrometers, including all values and ranges therein, such as from 1 micrometer to 300 micrometers, 100 micrometers to 300 micrometers, 300 micrometers to 1000 micrometers, and preferably in the range of 1000 micrometers to 2000 micrometers, once the rawhide shrinks upon drying. The pins may, therefore, be in the range of 0.1 % to 50 % larger in dimension that the desired pore size, including all values and ranges therein. The pins may exhibit a number of cross-sectional geometries.

Preferably, in embodiments, the pins and pores produced by them exhibit multiple arms to form an asterisk- or star- like geometry, including three or more arms and preferably from four to six arms. In other embodiments, the pins are preferably circular in cross-section but may alternatively be rectangular, triangular, ellipsoid quatrefoil or square in cross-section, or may be a combination of one or more of any of the shapes noted above. In any of the above embodiments, the pins are hollow. A cross-section of an example of a pin 200 exhibiting six arms and the pore 202 produced by such a pin are illustrated in FIGS. 2a and 2b. The micropore is illustrated in FIG. 2b as exhibiting a longest linear cross-sectional length, CL, which in the case of a circular pore is the pore diameter.

The micropores may be spaced uniformly, or randomly, over the surface of the rawhide at a density in the range of 1 to 100 pores per square centimeter (cm²), including all values in the range of 20 to 50 pores per square centimeter and more preferably in the range of 2 to 20 pores per square centimeter. In embodiments, pore density remains constant over the entire sheet. Alternatively, pore density is varied across the surface of a sheet such that the density increases or decreases across the sheet surface.

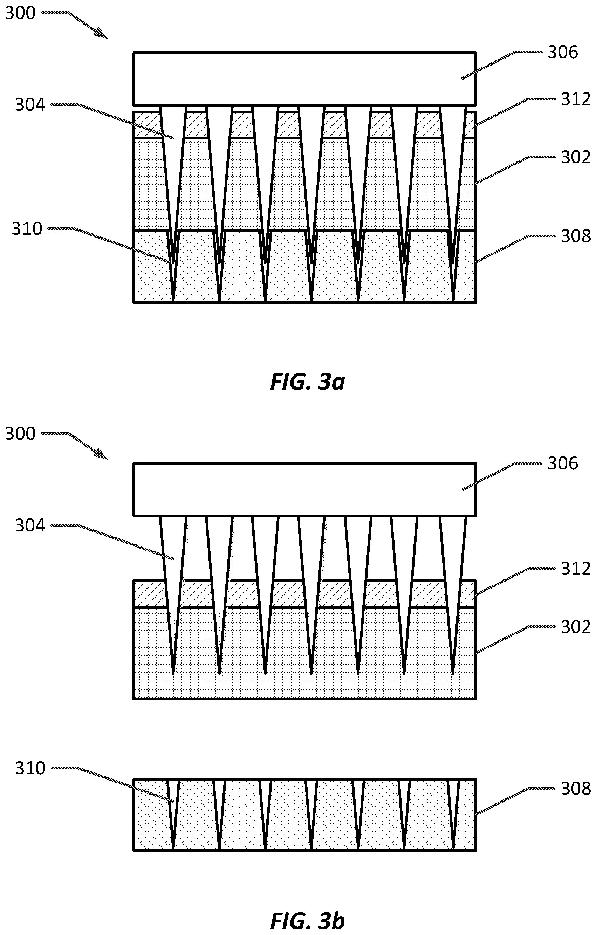

Generally, the pins are forced through the rawhide by utilizing equipment that may provide sufficient pressure to pierce the rawhide with the pins. As noted above, examples of such equipment include presses or calendaring rolls that may be operated using hydraulics, mechanical linkages or pneumatics. FIG. 3 illustrates an embodiment that utilizes a press 300. The press includes two plates 306 and 308 between which the rawhide 302 is placed. The plates are forced together to provide an elevated pressure of greater than 2 kPa, such as in the range of 2 kPa to 100 kPa, including all values and ranges therein, on the rawhide and pins. One plate, the carrier plate 5 306, carries the pins 304, and the other plate, the receiving plate 308, is the plate upon which the pins bear or are received. As illustrated in FIG. 3, the receiving plate may include a plurality of holes 310 for receiving the pins therein. In addition to applying force to pierce the rawhide, when the pins are received in the receiving plate 308, the plates may close together and squeeze excess water from the hide reducing the amount of water in the hide. The holes 310 may provide drain passages to facilitate water drainage out of the press. In alternative embodiments, both plates include both pins and holes. Further, in any of the above embodiments, the pins may retract into the carrier plate to facilitate stripping of the hide from the pins and the plate, or a stripper plate 312, as illustrated in FIG. 3, may be provided to help remove the rawhide from the pins once the press opens.

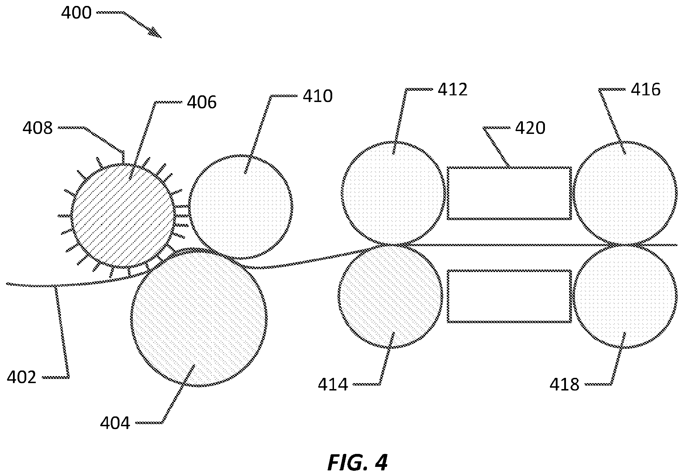

FIG. 4 illustrates an embodiment of calendaring equipment 400, wherein the rawhide 402 passes through a series of rolls. The rolls include at least one bearing roll 404 and at least one pin roll 406 on which a plurality of pins 408 are mounted. In addition, a squeeze roll 410 may optionally be provided. The pins 408, being carried by the pin roll 406, bear against the bearing roll 404 and pierce the rawhide 402 as the rawhide passes between the rolls. Then the squeeze roll 410, which is preloaded against the bearing roll 404 or an additional bearing roll (not illustrated), may be used to remove excess water from the rawhide. In embodiments, the pins may be retracted to facilitate release from the pin roll 406.

In embodiments, upon or after forming the micropores in the rawhide, the pores are at least partially filled or completely filled with chitosan. In addition, the surface between the pores may be coated with chitosan. Reference to completely filled micropores is reference to the feature that the micropores are filled with a sufficient amount of chitosan so that the filling is even or flush to the rawhide surface. Chitosan is reference to a polysaccharide composed of randomly distributed β-(1→4)-linked D-glucosamine (deacetylated unit). It is preferably sourced by deacetylation of chitin to about 50% of the free amine form. It may therefore have a heterogeneous type chemical structure made of both 1→4 linked 2-acetamido-2-deoxy-β-D-glucopyranose as well as 2-amino-2-deoxy- β-D-glucopyranose. Such heterogenous type chemical structure is illustrated below:

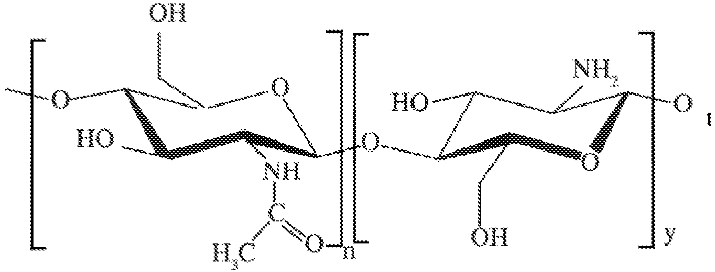

The chitosan offers a number of benefits. First, the chitosan itself is contemplated to provide antimicrobial activity, thereby serving to better preserve the rawhide after drying from microbial attack. In addition, as chewed upon by the animal, the chewed portions of the rawhide, which are contemplated then to have a greater susceptibility to microbial attack, are better preserved and the chewed portions are contemplated to be less likely to then mold and discolor. Second, as a polymeric material, the chitosan also can serve to prevent the pores, during drying of the rawhide, from closing. That is, the chitosan can also prevent the pores from "healing" themselves during drying so that the mircropores remain relatively intact. Accordingly, when ingested by the animal, the pores can still provide an increased surface area for exposure to digestive enzymes.

The chitosan may preferably be introduced into the pores and or onto the surface of the rawhide in a number of ways. Chitosan, as a solid, may be preferably dissolved into water solution and then sprayed onto the rawhide pores. The chitosan water solution may preferably contain 0.1 % by weight to 5.0% by weight chitosan. Such water solution may preferably be formed by mixing chitosan in water along with relatively small amounts of an acid, such as acetic acid. Such water solution of chitosan may therefore be delivered to the surface of the rawhide containing the micropores, by, e.g., spraying, and upon evaporation, can provide a film of chitosan within the pores. In addition, one may form a film layer of chitosan along all or a portion of the surface of the rawhide. The chitosan film thickness, whether within the micropore, or on the surface of the rawhide, is contemplated to have a thickness in the range of 1 micrometer to 2000 micrometers, or 1 micrometer to 1000 micrometers, or 1 micrometer to 500 micrometers, or 1 micrometer to 250 micrometers, or 1 micrometer to 100 micrometers.

As noted, preferably, the micropores are at least partially filled with chitosan and/or completely filled with chitosan, depending upon the particular final level of chitosan that one may wish to achieve for the rawhide sheet that is being treated.

As the pet chews upon the rawhide, the chitosan is loosened from the animal chew and is readily ingested by the animal. The chitosan is loaded into the micropores through pins that are hollow and injected upon pore formation. In some examples, chitosan may be pressed into the pores through further calendering of the rawhide sheets.

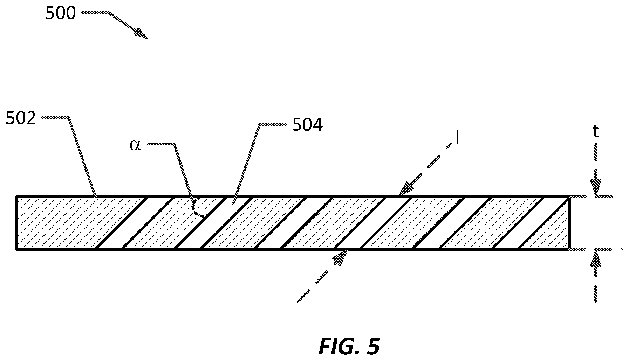

In addition, in any of the embodiments described above, the pins are preferably inserted into the rawhide at angle α relative to the surface 502 of the rawhide 500, thereby creating micropores having a length, 1, greater than the average thickness, t, of the rawhide as illustrated in FIG. 5. The angle α, relative to the surface of the rawhide, may be in the range of 10 to 80 degrees, including all values and ranges therein such as 30 to 60 degrees. Providing the pores at an angle may assist in increasing the surface area to volume ratio as well as the surface area available for contact with digestive juices. The angle α is constant or, alternatively, varied over the surface of the sheet. As illustrated, the surfaces of the rawhide may generally be parallel. However, it should be appreciated that the thickness of the rawhide sheets may vary.

Again referring to FIG. 1, the rawhide sheets may be formed into a desired final shape and dried 110 after the pores are formed. The sheets may be dried from 3 to 6 days at temperatures in the range of 35 °C to 80 °C, including all values and ranges therein. In embodiments, drying may occur under tension using a tension frame after removing the rawhide from a press or calendaring line. In other embodiments, as illustrated in FIG. 4, tenter rollers 412, 414, 416, 418 are placed in the process line after calendaring as illustrated in FIG. 4. To maintain a desired tension, the tentering rolls may rotate at faster speeds than the rolls which precede them. If other geometries are desired, the sheets may be cut, rolled and formed into rolls, rings, pretzels, sticks, braids, or chips. The rawhide may also be knotted to assume the general geometry of a bone or knotted bone. In alternative embodiments, the rawhide sheet may be die cut into desired shapes.

Once formed, the rawhide may then be dried with or without the assistance of a heat source such as an oven within the drying temperatures and times noted above. FIG. 4 illustrates the incorporation of an oven 420 in the process line, to facilitate drying of a sheet. The amount of water may be reduced to 1 to 20 % by weight of the final product, including all values and ranges therein.

The above, therefore, provides a rawhide animal chew formed from a rawhide sheet having a thickness in the range of 0.1 millimeters to 3.0 millimeters. The rawhide sheet includes a plurality of micropores having a longest linear dimension across the cross-section length, CL, in the range of 1 micrometer to 2000 micrometers and present at a density in the range of 1 pore to 100 pores per square centimeter. The pores assume one or more geometries, depending on the pin utilized to form the pores. In embodiments, the rawhide is a rawhide resin composition. The micropores, in embodiments, are at least partially and/or completely filled with chitosan and the surface of the rawhide is partially and/or fully coated with chitosan. The chitosan, which is preferably in film form within the pores and/or on the sheet of the rawhide, is mechanically retained in the pores due to shrinking of the rawhide as it dries. The rawhide sheet may be manipulated to assume the geometry of a roll, ring, pretzel, or knotted bone or die cut to provide a desired geometry.

The micropores in the rawhide sheet may extend completely through and/or only partially through the rawhide sheet. Accordingly, the micropores may be open on two sides of the rawhide sheet of open only on one side. As alluded to above, the pores may have a longest linear dimension across a cross-section of the micropores in the range of 1 micrometer to 2,000 micrometers. In the case of a round shaped micropores, such may correspond to the diameter of the pores. The pores may also have a thickness or penetration depth into the rawhide of 0.1 millimeters to 3.0 millimeters, or 0.1 millimeters to 2.0 millimeters, or 0.1 millimeters to 1.0 millimeters, or 0.1 millimeters to 0.5 millimeters. By way of example, for a rawhide sheet having a thickness of 0.1 millimeters to 3.0 millimeters, the thickness or penetration depth of the micropores may be equal to such value (0.1 millimeters to 3.0 millimeters), in which case the micropores would be open on both sides of the sheet. Alternatively, the micropores may have a penetration depth that is less than the full thickness of the rawhide sheet and therefore be open on only one side of the rawhide sheet.

An evaluation has been made using an in vitro procedure with simulated gastric and small intestine digestive fluids. Reference is made to the in vitro testing procedures reported by Boisen and Eggum, 1991, Nutr. Res. Rev. 4 141-162. Samples were incubated for 6 hours in simulated gastric fluid containing hydrochloric acid and pepsin, then for 18 hours in simulated small intestinal fluid containing pancreatin. Following incubation, percentage in vitro dry matter disappearance was calculated. Dimensions and weights of each treat were measured before and after incubation. Table 1 provides the results for samples of rawhide having different pore sizes and different spacing between the pores in the identified samples:

**Table I.**

| **Small Intestine Dry Matter Disappearance** | |
|---|---|
| **Sample** | **Intestinal Phase (18 Hours)** |
| | **% Dry Matter Disappearance** |
| **I (Control-No Pores)** | **70.2** |
| **II (2.0 mm pores/6.0 mm apart)** | **84.3** |
| **III (1.0 mm pores/3.0 mm apart)** | **92.1** |

The microporous animal chews herein is one that is capable of indicating an intestinal phase dry matter disappearance in small intestinal fluid containing pancreatin, of 84.3 % and as high as 92.1 %, as compared to a control value of 70.2%. The average thickness of these tested samples was 1.7 mm. Accordingly, in preferred embodiment, it is contemplated that the microporous animal chews herein indicate a dry matter disappearance in simulated small intestinal fluid containing pancreatin in the range of greater than 70.2 % to 95.0%, more preferably in the range of 75.0% to 95.0%, even more preferably 80.0% to 95.0%, and in a most preferred embodiment, in the range of 85.0% to 95.0 % or 90.0% to 95.0 %.

In particular, for 1.0 mm pores, where the pore size may range from 0.90 mm to 1.1 mm, that are spaced 3.0 mm apart (+/- 0.5 mm) it was considered remarkable that one could achieve for such pore size, after 18 hours in the identified testing environment, a % dry matter disappearance of greater than 90% in small intestinal fluid containing pancreatin, and as noted, in the range of 90.0 % to 95.0 %. Moreover, it is contemplated here that when the pore size is reduced below 1.0 mm, and falls in the range of 0.001 mm to 0.99 mm, after 18 hours in the identified testing environment, the % dry matter disappearance will fall in the range of greater than 70.2% up to 100%. In addition, it is similarly contemplated that when one reduces the thickness below the average thickness of the samples tested (1.7 mm), to a thickness of 0.1 mm to less than 1.7 mm, the % dry matter disappearance will similarly fall in the range of greater than 70.2% up to 100%.

The foregoing description of several methods and embodiments has been presented for purposes of illustration. It is not intended to be exhaustive or to limit the claims to the precise steps and/or forms disclosed, and obviously many modifications and variations are possible in light of the above teaching. It is intended that the scope of the invention be defined by the claims appended hereto.

## Claims

1. A method of forming a microporous animal chew, comprising:
providing a wet rawhide sheet having a thickness in the range of 0.5 millimeters to 4.0 millimeters including water present at 60 % to 80 % by weight of the total weight of the rawhide sheet or greater;
piercing said rawhide sheet with hollow pins (304, 408) and forming micropores (108) in said rawhide sheet, wherein said micropores (108) have a largest linear cross-sectional length in the range of 1 micrometer to 2,000 micrometers and are arranged to provide a pore density in the range of 1 to 100 pores per square centimeter;
partially filling said micropores (108) with chitosan by injecting the chitosan into the micropores (108) through the hollow pins upon micropore (108) formation; and
drying said rawhide sheet, wherein said rawhide sheet includes 1 to 20 % by weight water of the total weight of the rawhide sheet.

2. The method of claim 1, further comprising
- removing a portion of water present in said rawhide sheet prior to drying or
- stretching said rawhide sheet prior to drying or
- filling said micropores (108) with a support additive prior to drying.

3. The method of claim 1, wherein said rawhide sheet is provided between a carrier plate (306) and a receiving plate (308), wherein said carrier plate (306) carries said pins (304), and piercing said rawhide sheet includes forcing said plates (306, 308) together.

4. The method of claim 3, wherein said receiving plate (308) includes holes (310) to receive said pins (304) therein.

5. The method of claim 3, further comprising compressing said rawhide sheet between said plates (306, 308) to remove at least a portion of water present in said rawhide sheet.

6. The method of claim 1, wherein said rawhide sheet is fed into calender rollers, wherein one of said rollers includes said pins (408) and said pins (408) pierce said rawhide sheet as said pins (408) bear against a bearing roll (404).

7. The method of claim 6, further comprising compressing said rawhide sheet between a squeeze roll (410) and said bearing roll (404) and removing a portion of said water present in said rawhide sheet.

8. The method according to claim 7, further comprising stretching said rawhide sheet prior to drying by passing said rawhide sheet between tenter rolls (412, 414, 416, 418).

9. The method of claim 1, wherein said pins (304, 408) pierce said rawhide sheet at an angle α relative to a surface (502) of said rawhide (500), wherein α is in the range of 10 to 80 degrees.

10. The method of claim 9, wherein said micropores (108) exhibit a length that is greater than the thickness of said rawhide sheet.

11. The method of claim 1 wherein said wet rawhide sheet includes a rawhide resin composition.

12. The method of claim 1, wherein said dried rawhide has a thickness of 0.1 millimeters to 3.0 millimeters.

## Patentansprüche

1. Verfahren zur Herstellung eines mikroporösen Tierkaukörpers, umfassend:
Bereitstellen einer feuchten Rohhautbahn mit einer Dicke im Bereich von 0,5 Millimeter bis 4,0 Millimeter, die Wasser in einer Menge von 60 bis 80 Gew.-% des Gesamtgewichts der Rohhautbahn oder mehr enthält;
Durchstechen der Rohhautbahn mit Hohlstiften (304, 408) und Bilden von Mikroporen (108) in der Rohhautbahn, wobei die Mikroporen (108) eine größte lineare Querschnittslänge im Bereich von 1 Mikrometer bis 2.000 Mikrometer aufweisen und so angeordnet sind, dass sie eine Porendichte im Bereich von 1 bis 100 Poren pro Quadratzentimeter ergeben;
teilweises Füllen der Mikroporen (108) mit Chitosan durch Injizieren des Chitosans in die Mikroporen (108) durch die Hohlstifte nach der Bildung der Mikroporen (108); und
Trocknen der Rohhautbahn, wobei die Rohhautbahn 1 bis 20 Gew.-% Wasser, bezogen auf das Gesamtgewicht der Rohhautbahn, enthält.

2. Verfahren nach Anspruch 1, das ferner umfasst
- Entfernen eines Teils des in der Rohhautbahn vorhandenen Wassers vor dem Trocknen oder
- Dehnen der Rohhautbahn vor dem Trocknen oder
- Füllen der Mikroporen (108) mit einem Stützadditiv vor dem Trocknen.

3. Verfahren nach Anspruch 1, wobei die Rohhautbahn zwischen einer Trägerplatte (306) und einer Aufnahmplatte (308) angeordnet ist, wobei die Trägerplatte (306) die Stifte (304) trägt, und wobei das Durchstechen der Rohhautbahn ein Zusammenpressen der Platten (306, 308) umfasst.

4. Verfahren nach Anspruch 3, wobei die Aufnahmeplatte (308) Löcher (310) aufweist, um die Stifte (304) darin aufzunehmen.

5. Verfahren nach Anspruch 3, das ferner ein Zusammenpressen der Rohhautbahn zwischen den Platten (306, 308) umfasst, um zumindest einen Teil des in der Rohhautbahn vorhandenen Wassers zu entfernen.

6. Verfahren nach Anspruch 1, wobei die Rohhautbahn in Kalanderwalzen eingeführt wird, wobei eine der Walzen die Stifte (408) aufweist und die Stifte (408) die Rohhautbahn durchstechen, wenn die Stifte (408) gegen eine Lagerwalze (404) drücken.

7. Verfahren nach Anspruch 6, das ferner ein Zusammendrücken der Rohhautbahn zwischen einer Quetschwalze (410) und der Lagerwalze (404) und ein Entfernen eines Teils des in der Rohhautbahn vorhandenen Wassers umfasst.

8. Verfahren nach Anspruch 7, das ferner ein Strecken der Rohhautbahn vor dem Trocknen durch ein Führen der Rohhautbahn zwischen Spannwalzen (412, 414, 416, 418) umfasst.

9. Verfahren nach Anspruch 1, wobei die Stifte (304, 408) die Rohhautbahn in einem Winkelα relativ zu einer Oberfläche (502) der Rohhaut (500) durchstechen, wobei α im Bereich von 10 bis 80 Grad liegt.

10. Verfahren nach Anspruch 9, wobei die Mikroporen (108) eine Länge aufweisen, die größer ist als die Dicke der Rohhautbahn.

11. Verfahren nach Anspruch 1, wobei die nasse Rohhautbahn eine Rohhaut-Harzzusammensetzung enthält.

12. Verfahren nach Anspruch 1, wobei das getrocknete Rohleder eine Dicke von 0,1 Millimetern bis 3,0 Millimetern aufweist.

## Revendications

1. Procédé de fabrication d'un os à mâcher microporeux pour animaux, comprenant les étapes consistant à :
utiliser une feuille de cuir brut humide ayant une épaisseur s'inscrivant dans la plage de 0,5 millimètre à 4,0 millimètres comprenant de l'eau présente à au moins 60 % à 80 % en poids du poids total de la feuille de cuir brut ;
percer ladite feuille de cuir brut à l'aide d'aiguilles creuses (304, 408) et former des micropores (108) dans ladite feuille de cuir brut, dans lequel lesdits micropores (108) ont une longueur de section transversale linéaire la plus grande s'inscrivant dans la plage de 1 micromètre à 2000 micromètres et sont prévus pour fournir une densité de pores s'inscrivant dans la plage de 1 à 100 pores par centimètre carré ;
remplir partiellement lesdits micropores (108) de chitosane en injectant le chitosane dans les micropores (108) par l'intermédiaire des aiguilles creuses lors de la formation de micropores (108) ; et
sécher ladite feuille de cuir brut, dans lequel ladite feuille de cuir brut comprend de 1 à 20 % en poids d'eau du poids total de la feuille de cuir brut.

2. Procédé selon la revendication 1, comprenant en outre les étapes consistant à :
- éliminer une partie de l'eau présente dans ladite feuille de cuir brut avant le séchage ou
- étirer ladite feuille de cuir brut avant le séchage ou
- remplir lesdits micropores (108) d'un additif de support avant le séchage.

3. Procédé selon la revendication 1, dans lequel ladite feuille de cuir brut est disposée entre une plaque porteuse (306) et une plaque de réception (308), dans lequel ladite plaque porteuse (306) porte lesdites aiguilles (304), et l'étape de perçage de ladite feuille de cuir brut consiste à forcer lesdites plaques (306, 308) l'une contre l'autre.

4. Procédé selon la revendication 3, dans lequel ladite plaque de réception (308) comprend des trous (310) servant à recevoir lesdites aiguilles en leur sein.

5. Procédé selon la revendication 3, comprenant en outre l'étape consistant à compresser ladite feuille de cuir brut entre lesdites plaques (306, 308) pour éliminer au moins une partie de l'eau présente dans ladite feuille de cuir brut.

6. Procédé selon la revendication 1, dans lequel ladite feuille de cuir brut est amenée dans des rouleaux presseurs, dans lequel l'un desdits rouleaux comprend lesdites aiguilles (408) et lesdites aiguilles (408) percent ladite feuille de cuir brut lorsque lesdites aiguilles (408) appuient contre un rouleau d'appui (404).

7. Procédé selon la revendication 6, comprenant en outre l'étape consistant à compresser ladite feuille de cuir brut entre un rouleau exprimeur (410) et ledit rouleau d'appui (404) et à éliminer une partie de l'eau présente dans ladite feuille de cuir brut.

8. Procédé selon la revendication 7, comprenant en outre l'étape consistant à étirer ladite feuille de cuir brut avant le séchage en faisant passer ladite feuille de cuir brut entre des rouleaux d'étirage (412, 414, 416, 418).

9. Procédé selon la revendication 1, dans lequel lesdites aiguilles (304, 408) percent ladite feuille de cuir brut à un angle α par rapport à une surface (502) dudit cuir brut (500), dans lequel l'angle α s'inscrit dans la plage de 10 à 80 degrés.

10. Procédé selon la revendication 9, dans lequel lesdits micropores (108) ont une longueur qui est supérieure à l'épaisseur de ladite feuille de cuir brut.

11. Procédé selon la revendication 1, dans lequel ladite feuille de cuir brut comprend une composition de résine de cuir brut.

12. Procédé selon la revendication 1, dans lequel ledit cuir brut séché a une épaisseur de 0,1 millimètre à 3,0 millimètres.
